# EUROPEAN PATENT APPLICATION

(11) **EP 2 851 850 A1**
(43) Date of publication of application: **25.03.2015**
(21) Application number: 13772828.3
(22) Date of filing: 29.03.2013
(51) Int. Cl.: G06N 3/00

(54) **LOGIC ELEMENT COMPLEX BASED ON BIOMOLECULES (VARIANTS)**

(30) Priority: 02.04.2012 RU 2012112643
(71) Applicant: Nikitin, Maxim Petrovich, Moscow 127562 (RU)
(72) Inventor: Nikitin, Maxim Petrovich, Moscow 127562 (RU)
(74) Representative: Hoffmann Eitle
(86) International application number: PCT/RU2013/000269
(87) International publication number: WO 2013/151465

(57) **Abstract**

The invention relates to the field of logic elements, specifically to logic elements based on biomolecules. The essence of the invention consists in a logic element complex which converts input signals into an output action according to a given logic function. Depending on the output action, the proposed logic element complex can be used both for computational purposes and for various applications in biomedicine, e.g., for diagnostics or therapy of diseases, targeted delivery of a substance to target cells, etc. The technical result when using the invention consists in a logic element complex, for which a plurality of input signals and output actions can be virtually unlimited, said signals and output actions can be different in nature, and, in addition, implementation of a wide range of logic functions for the same input signals is made possible.

## Description

The invention relates to the fields of logic elements, namely to the field of logic elements based on biomolecules. The invention allows conversion of input signals into output action according to a given logic function. Depending on the output action, the proposed logic element complex may be used for both computational purposes and for various biomedical applications, for example, for diagnostics or therapy of diseases, targeted delivery of a substance to target cells, etc.

Biological computing systems are of great interest both as computing technics and for various fields of medicine and biology. From the point of view of computing technics, the efficiency of biological computer systems may be higher as compared with traditional computing systems due to ability of realization not only binary but also multi-valued logic (for example, if a variable is an oligonucleotide consisting of 20 bases, the multi-valuedness may reach 4²⁰, which corresponds to about 10¹² bits). Moreover, the computing system capable of operating with these oligonucleotides located in the same solution is, in fact, a quantum computer. Currently, computing systems based on oligonucleotides are just beginning to develop. In other areas, such as medicine, computing systems of a different kind are required. For example, the systems would be extremely useful that would comprise a computing unit checking a presence or absence of various signals from the environment and also an action unit, which, depending on the result from the computing module might perform some biologically meaningful action, for example, kill a cancer cell in an organism. Such systems require transmission of information between the computing and action units. It is clear that the wider the range of possible signals of a computer system and the range of actions of the action unit, the better. Such actions should not necessarily be due to the presence of some molecules, but due to, for example, an electromagnetic radiation (including light and low-frequency field), a change of pH, temperature, etc.

The biological computing systems exist and are widespread that are capable of performing the aforementioned functions under a sole condition regarding one input, for example, "kill the cell if it carries on its surface a specific marker" (an antibody with a radioactive isotope can be an example of such a system). Besides, there are systems that recognize two effects as inputs, for example, "kill a cell if the medium has a decreased level of pH and the cell carries on its surface a specific marker." However, such multiple-input systems cannot be easily reorganized to perform any desired function of variables. For instance, said systems cannot be easily be reorganized to implement negation of an input or its output action, e.g., "kill a cell if it does not carry on its surface a specific marker" or "do not kill a cell if it carries on its surface a specific marker" (although from a viewpoint of mathematical logic these expressions are identical, they may differ in biology because killing cells is a statistically quantitative feature rather than binary-qualitative - kill/not kill, and the abovementioned functions may have substantially different nonlinearity of the action depending on the presence of the marker). A method is known (U.S. Patent 7,745,594 B2, issued June 29, 2010), in which a logic gate is an oligonucleotide sequence where logic gates are implemented on input oligonucleotides due to branch migration of DNA comprised in the logic element. Different logic functions are realized by different sequences of oligonucleotides.

The drawbacks of this known method are as follows:
1) As inputs, only oligonucleotides (or their variants) can be used. This approach is not applicable to molecules of other nature such as carbohydrates, proteins, low-molecular organic compounds, etc.
2) The time of signal transmission is extremely long due to low rate of displacement of one oligonucleotide by another oligonucleotide.
3) The output signal can be only release of an oligonucleotide from the logic element complex.

A method is known (Patent application WO2011116151 (A2) of September 22, 2011), in which a sequence of enzymes is used for biocatalytic reaction that is a Boolean logic function of received "input" signals of biomarkers. A binary signal is generated, and the signal that equals 1 means the presence of a disease or a trauma.

The drawbacks of this known method are as follows:
1) As input signals, only substrates, co-factors or products of the used enzymes can be used. When using enzymes, the number of combinations is substantially limited.
2) As the basis of the logic element, enzymes are used, the variety of which is very limited.

A method is known (Nanoparticle of Self-Assembly Gated by Logical Proteolytic Triggers, g. von Maltzahn, et al., J Am Chem Soc, 2009) that is most similar to the proposed method, wherein two particles assemble together when an enzyme breaks a bond between a particle and polyethyleneglycol that masks neutravidin on one particle and biotin on the other one.

The drawbacks of this known method are as follows:
1) As the input signals, enzymes are used.
2) The bond between the particles and the masking polyethyleneglycol is covalent.
3) Solely AND and OR logical functions can be implemented.
4) The output signal of the logic element is limited to the states of aggregated particles (the output value of the Boolean function is TRUE) and to colloidally stable particles (the output value of the function is FALSE).

Thus, the required technical result consists in creation of a logical element for which the range of input signals, as well as of output actions could be virtually unlimited, and said signals and actions could be diverse in nature, and, besides, it would be possible to implement a wide range of logical functions for the same inputs.

To achieve this technical result, a logic element complex is proposed that converts input signals into an output action according to a given logic function and comprises at least:
i) an agent (a molecule, a particle, a surface of a solid phase) having at least a linking receptor 1 and a linking receptor 2,
ii) a blockable label, participating directly or indirectly in producing at least one output action and capable of binding directly or indirectly with said linking receptor 2 of said agent so that the binding of said blockable label with said linking receptor 2 of said agent is governed by at least one input signal,
iii) a blocking substance, which is capable of binding directly or indirectly with said linking receptor 1 of said agent depending on at least one of the input signals, and such that upon binding of said blocking substance with said linking receptor 1 of said agent, said blockable label, which under respective input signals is bound with said agent, becomes blocked spatially or spatially-electrostatically, and this blocking causes a change in said output action.

Besides, a logic element complex, wherein said output action is produced by said blockable label of said agent depending on a direct or indirect interaction of said blockable label with an object, said output action being different from binding of the agents with each other.

Besides, a logic element complex, which converts input signals into an output action according to a given logic function and comprising at least:
i) an agent (a molecule, a particle, a surface of a solid phase) having at least a linking receptor 1 and a blockable label that participates directly or indirectly in producing at least one output action,
ii) a blocking substance, which is capable of binding directly or indirectly with said linking receptor 1 of said agent depending on at least one of the input signals, and such that upon binding of said blocking substance with said linking receptor 1 of said agent, said blockable label of said agent becomes blocked spatially or spatially-electrostatically, and this blocking causes a change in said output action,
   and said output action is implemented by said blockable label of said agent depending on a direct or indirect interaction of said blockable label with an object, said output action being different from binding of the agents with each other.

Besides, a logic element complex wherein said output action is produced by said blockable label of said agent depending on a direct or indirect interaction of said blockable label with either a surface of a solid phase (including the surface of a cell) or an object that produces said output action or changes the output action produced by the blockable label, the object being different from the agent (or its analog).

Besides, a logic element complex wherein at least one of said agent or said blocking substance is a magnetic, fluorescent, protein (including cross-linked protein), polymer (polystyrene, dextran, polypeptide, etc.) or crystalline (gold, silver, semiconductor, etc.) nanoparticle or microparticle.

Besides, a logic element complex, wherein said blockable label is an enzyme.

Besides, a logic element complex wherein said blocking of said blockable label causes suppression (reduction), at least partial, of said output action.

Besides, a logic element complex, wherein at least one of said dependences of binding of the blocking substance or the blockable label with the agent upon the input signal of molecular nature is due to the presence of the "receptor to said input signal - said input signal (or its analog)" bond in a series of bonds between said agent and said blocking substance, or said agent and said blockable label.

Besides, a logic element complex wherein the process of producing of said output action involves a specific direct or indirect binding with said blockable label of at least one molecule or particle, which is a label capable of generating the detectible signal, preferably fluorescent, luminescent, enzyme, radioactive, magnetic, or exhibiting surface plasmon resonance properties.

Besides, a logic element complex, wherein after binding of said molecule or particle with said blockable label of said agent, a major portion of unbound said molecules or particles is removed (separated), and said output action is produced primarily by said molecules or particles bound with said agent.

Besides, a logic element complex, wherein the process of producing of said output action involves a specific direct or indirect binding with said blockable label of at least one molecule or particle, immobilized on an auxiliary solid phase (an immunochromatographic test strip, a plastic multi-well plate, etc.), and for this purpose a liquid medium containing said logic element complex is passed along said solid phase or incubated in contact with said solid phase, and said output action is associated with the number of the agents bound with said auxiliary solid phase.

Besides, a logic element complex, wherein said output action consists in at least generation of a detectible signal.

Besides, a logic element complex, wherein said output action is designated for diagnostics *in vitro* of a disease or condition (state) of a subject.

Besides, a logic element complex, wherein said output action is designated for diagnostics *in vivo* of a disease or condition of a subject.

Besides, a logic element complex, wherein said output action is designated for therapeutic influence on health or condition of a subject.

Besides, a logic element complex, which is used as a basis for a pharmaceutical formulation.

Besides, a logic element complex, wherein at least a part of said input signals is governed by condition of a subject.

Besides, a logic element complex, wherein the blockable label is a receptor (an antibody to a marker on a cell membrane, nuclear localization signal peptide, etc.) for targeted delivery of a substance to the area of interest in the organism, a cell, etc.

Besides, a logic element complex, wherein the output action consists in creation of molecules for improvement of health of a subject or for diagnostics of its (his/her) condition.

Besides, a logic element complex, which is made intended for administration (intravenously, subcutaneously, etc.) to a subject with a purpose of disease treatment (therapy) or diagnostics of condition of the subject.

Besides, a logic element complex, wherein the output action consists in delivery of a substance to target cells.

Besides, a logic element complex, wherein said output action is produced by said blockable label of said agent depending on direct or indirect interaction of said blockable label with an object, which does not lead to specific binding of two agents.

Besides, a logic element complex, wherein said output action is produced by said blockable label of said agent depending on a direct or indirect interaction of said blockable label with other molecules or particles, or surfaces of solid phase (including the surface of cells) excluding those located on other agents that are identical or similar to said agent (i.e., those having other blockable labels and/or linking receptors).

Besides, a logic element complex, wherein said output action is produced by said blockable label of said agent depending on direct or indirect interaction of said blockable label with an object, said object being chosen different from the agent and not similar to the agent; and the producing said output action is significantly affected by the number of interactions between said blockable label and said signal object and is insignificantly affected by the fact, whether each said object interacts with several agents or merely one agent.

Besides, a logic element complex, wherein said output action does not depend substantially upon aggregation of the agents with each other.

Besides, a logic element complex, wherein the contribution of said blockable label in producing said output action consists in interaction with an object, said object being chosen different from the agent and not similar to the agent; and the producing of said output action is significantly affected by the number of "blockable label-said object" interactions and is insignificantly affected by the fact, whether said object interacts with several said agents or merely one said agent (when a molecule or a particle, not a surface of solid phase, nor a surface of a cell is chosen as said object).

All variants of the invention described below are given merely for illustration of diversity of possible embodiments of the invention, not as a limitation.

The agent can be any molecule, nanoparticle or microparticle, or a surface of a solid phase with which other molecules can be bound in any way. For example, this may be a molecule of protein, glycoprotein, a magnetic, gold, plastic, crystal, protein, etc. nanoparticle or microparticle. The surface of solid phase may be any standard surface used in laboratory practice, e.g., plastic multi-well plates or immunochromatographic test strips, or plastic test tubes.

The input signal that affects the binding of blocking substance and the linking receptor 1 of the agent may be effects different by nature. Besides, in one embodiment of the invention, the same effects may influence similar bonds of the blockable label and the linking receptor 2 of the agent.

For example, the input may be an electromagnetic radiation (e.g., light), and the bond between the blocking substance and the linking receptor 1 may break (or, conversely, form) under effect of this radiation. The intensity, at which the majority of said bonds break, may be regarded as the value for a variable (input) equal to 1, while less intensity - equal to 0. Besides, if this bond is destroyed by radiation at a certain frequency, then it is better to consider the bond as a binary variable (the bond is available or not, i.e., 1 or 0), or assume that the radiation can take not only a binary value, but also be multi-valued, for example, be a function of intensity on frequency). Besides, more complicated situations can be discussed where complex optical effects are used, such as two-photon absorption and others, when an additional parameter appears. It is clear that such cases only extend the capabilities of the system.

If the input signal is a value of pH or temperature, different labile bonds can be broken at different values of pH or temperature, and, again, it makes more sense to speak about a value of the variable related to the bond, not to the input stimulus. On the other hand, pH and temperature have only one parameter, unlike the electromagnetic radiation which, as indicated above, has two parameters: frequency and intensity. Accordingly, the variables corresponding to the bonds are not linearly independent. At the same time, in certain cases it is more convenient to consider pH and temperature as multi-valued, not binary, variables.

Besides, the input signal may be a particle, molecule, ion or atom that either contribute to formation of a bond (including mediation of it), or break some bond, for example, the bond between said linking receptor 1 and said blocking substance, or the bond between said linking receptor 2 and said blockable label.

The linking receptor may be, for example, any atom or a molecule that has a complementary molecule (ligand). In this case, the "linking receptor - ligand" pair may be any pair of specifically interacting molecules. The following pairs can be mention as examples, not as a limitation: antigen - antibody, oligonucleotide - oligonucleotide, lectin - carbohydrate, lectin - glycoprotein, streptavidin - biotin, protein A - immunoglobulin, enzyme - substrate, enzyme-activator-zymogen, etc. Within this description, both the first molecule and the second molecule of the mentioned pairs may be equally considered to be the ligand or receptor, i.e., for example, the ligand may be an antibody while the receptor - an antigen to said antibody, the ligand may be a lectin while the receptor - a corresponding carbohydrate, etc. For convenience of the description, the term "ligand" refers to the ligand itself, as well as to its analogs, i.e. any molecules or molecular complexes, and particles, whose part is homologous to the ligand, including molecular complexes. For example, the term "receptor to ligand" includes the term of a receptor to the ligand's analog. In one embodiment of the invention, a ligand of the linking receptor 1 can serve as the input signal and compete with the blocking substance for binding with the linking receptor 1 (in the format of either displacement of the blocking substance from the bond with the linking receptor 1, or inhibition of the linking receptor or the blocking substance so that the blocking substance cannot bind to the linking receptor 1), or realize binding of the blocking substance with the linking receptor 1.

However, for certain bonds, the input signal may differ from the ligand (or its analog) or the linking receptor 1, for example, if the linking receptor 1 is a nickel-nitrilotriacetic acid (or simply a nickel ion bound with the agent via a coordination bond), and the blocking substance binds with it via a polyhistidine tag, then the input signal may be, for example, a nickel ion or ethylenediaminetetraacetic acid, which displaces the blocking substance from the bond with the linking receptor 1.

Besides, for example, if the linking receptor 1 is a metal ion-dependent receptor (e.g., concanavalin A, which requires bivalent ions for binding of glucose), and the blocking substance carries glucose residues, the input signal can be ethylenediaminetetraacetic acid, which deprives concanavalin of metal ions, and, as a result, the bond will be broken.

As it is noted above, the bond between the linking receptor 1 and the blocking substance may also not be based on a specific molecular interaction. This may be any bond which may be either formed or broken by an external stimulus, the examples of which include electromagnetic radiation (e.g., photoactivatable cross-linking reactions), pH, temperature, the enzymatic reaction (cross-linking by various enzymes, such as ligases) and others. In this case, the input signal is the stimulus itself.

Besides, in one embodiment of the invention, said input signal can destroy, forbid or mediate not only the bond between the linking receptor 1 with the blockable label, but also the bond between the linking receptor 2 with the blockable label the same way as described above. It should be noted that sometimes in the description below, for convenience, only binding of the linking receptor 1 with the blockable substance will be mentioned, but, where appropriate, that discussion may also relate to binding of the linking receptor 2 with the blockable label.

The blockable label may be any atom, molecule or particle, the spatial or spatial-electrostatic blocking of which changes the output action. Examples of the blockable label can be any detectible label such as fluorescent (including atomic or ionic), radioactive, magnetic, exhibiting surface plasmon resonance properties and so on. In the case of a fluorescent label, its spatial blocking may consists in inability of the fluorescence quencher approach close enough to a fluorophore for its quenching or, on the contrary, the blockable label can be the fluorescence quencher, and its blocking may consist in inability of the fluorophore to approach the quencher. Besides, the blockable label may be one fluorophore (or a receptor labeled with such fluorophore) of a pair of fluorophores with possible Förster resonance energy transfer (FRET) between them. Then the blocking consists in inability of the components of this pair to approach each other. Besides, it may be an enzyme, the action of which can be detected. For example, this could be an enzyme, whose work transforms an uncolored substrate into a colored product, or non-fluorescent substrate into fluorescent product. Besides, this can also be an enzyme that triggers a cascade reaction, for example, its substrate is a zymogenic form of another enzyme, which, when activated, generates a detectible signal as a result of its work, etc. Besides, the blockable label may be any receptor that can bind another molecule so that the fact of binding with this molecule can be registered. For example, said molecule may be one of the abovementioned detectible labels. Besides, for example, this molecule may be immobilized on any surface or a particle, (for example, on a plastic multi-well plate or a immunohromatografic test strip); in this case, the detection of binding of said molecule and the blockable label is carried out using a marker bound to the blockable label. In the case of the immunohromatografic test strip, this label can be, for example, a colored polymer particle, magnetic or gold particle. In the case of the plastic plate, this may be, for example, an enzyme or fluorescent label. Besides, the role of this label may be performed by, inter alia, said agent or said molecules on the agent, or molecules contained in the agent.

Besides, the blockable label may represent a number of labels different in nature, for example, those mentioned above. Then the combined signal from the different labels may change the parameters of output action, for example, by increasing the sensitivity or expanding the dynamic operational range of the output action. These examples are given merely as illustration of possible nature of the blockable label, not as a restriction of the choice of variants of the blockable label.

Besides, the blockable label may produce an output action, which is not only generation of a detectible signal.

The output action may be any change in state of a system or of its part in the sense that it is possible to distinguish the state of the system without said action and under said action. Examples of the output action include a change in fluorescence of the system, or its radioactivity, or magnetic properties of the system, etc. "Emergence" of radioactivity can be accomplished, for example, as follows. Let an antibody be the blockable label. Upon unblocking of this blockable label, it can bind a radioactively labeled antigen. After washing of unbound antigen, radioactivity remains in the system. If the antibody cannot bind the antigen (binding is blocked), then after washing of unbound antigen the radioactivity level becomes much lower (trace signal of the unwashed antigen).

In one embodiment of the invention, the output action can be any detectible signal generated by a non-blocked blockable label and not generated (or generated to a lesser degree) by a blocked blockable label; the signal can be detected qualitatively (yes/no) or measured quantitatively, and then referenced with the value of a logic function. Detection may be accomplished by any known method such as optical, magnetic, electrochemical, etc. means of measurements.

In one embodiment of the invention, the output action can be defined by interaction of a blockable label with a ligand immobilized on a solid phase, e.g., on an immunochromatographic strip or a multi-well plate. After adding of input signals to the logic element complex and waiting for the time required for implementation of the logic element, a mixture containing said logic element complex is passed over the test strip (as in the conventional lateral flow method, immunochromatography or lateral flow) or is incubated in the plate. Then the signal proportional to the amount of the agents captured on the solid phase, i.e. the agents interacted with the immobilized ligand, is readout by any known method: either by an optical signal of the logic element complex or by a magnetic signal, or is manifested biochemically (using, for example, an enzymatic reaction) due to other receptors carried by the agent.

Besides, when the blockable label binds with the agent via the linking receptor 2 of the agent depending on the input, as well as when it is associated with the agent without the linking receptor 2, the output action can lead to aggregation of the agents. For example, upon addition to the logic element complex of a substance that interacts with unblocked, but bound to the agent blockable labels so that one molecule of said substance can bind simultaneously with at least two blockable labels belonging to the different agents, the aggregation of the agents occurs, which can be detected by any standard method. Another variant is implemented as follows. At least two agents are used, the blockable label of one agent being complementary to the blockable label of the second agent. Then the aggregation of the particles can be measured by any known method, e.g., by turbidimetric assay, or by a change of characteristics of the plasmon resonance if using gold nanoparticles as the basis for one or both particles, or by a change of the NMR (nuclear magnetic resonance) signal if using magnetic particles as the basis for one or both particles, or by other methods.

However, aggregation of the agents may not always be useful and sufficiently sensitive output action. When an output action should be produced, which can be detected with high sensitivity, the output action may need to be essentially different than the aggregation of the agents. In one embodiment of the invention, said output action is produced by said blockable label of said agent depending on direct or indirect interaction of said blockable label with an object that does not lead to association of the two agents, i.e. does not lead to their aggregation. In another embodiment of the invention, said output action is produced by said blockable label of said agent by means of direct or indirect interaction of said blockable label with said object that is not located on another agent; side interaction of the two agents not contributing substantially to the output action. This means that if, for example, a protein labelled by fluorescein binds with the blockable label (e.g., anti-fluorescein antibody), the output action is governed by fluorescence of fluorescein on the agent. When adding not too large excess of said protein labelled by fluorescein, a minor aggregation of the agents may occur due to the fact that said protein labelled by fluorescein can bind more than one agent. In this embodiment of the invention, the output action is governed by the ability of said protein labelled by fluorescein to bind with the agent and produce the fluorescent signal, for example, after washing out the molecules of said protein unbound to the agent, not by aggregation of the agents, though in this case minor aggregation of the agents may be also possible causing insignificant quenching of fluorescence. For example, in other embodiment of the invention, said output action is produced by said blockable label of said agent depending on direct or indirect interaction of said blockable label with the object, said output action being different from binding of the agents with each other. In other embodiment of the invention, said output action is produced by said blockable label of said agent depending on direct and indirect interaction of said blockable label either with a surface of a solid phase (including a suface of a cell) or with an object that produces said output action or changes the output action produced by the blockable label, said object being different from the agent (or its analog). This means that said interaction does not imply that the output action is governed merely by aggregation of the agents with each other. Even if the aggregation occurs, the output action is produced due to another phenomenon. For example, if the blockable label on the agent is a polyclonal antibody against bovine serum albumin (BSA), and said object is a bovine serum albumin labelled by fluorescein, then upon unlocking of the blockable label on the agent, the blockable label binds the fluorescein-labeled BSA from the solution. Then, since the blockable label is a polyclonal antibody, under low concentration of the labelled BSA several agents may bind to one molecule of the labelled BSA, and partial aggregation may happen. However, in this case it is much easier and more sensitive to detect (if the output action should generate a detectible signal) the fluorescein bound with the agents by its fluorescence rather than determine the degree of aggregation of the particles. Besides, under addition of significant excess of BSA labelled by fluorescein, specific binding of the agents via BSA does not happen and therefore the degree of aggregation of the agents decreases. At the same time, the fluorescent signal remains proportional to the number of unblocked blockable labels, not to the degree of aggregation of the agents. It should be noted that non-specific aggregation of the agents may occur and in practice happens in any case, i.e. when the input signals are available or not available, thus there are no processes that are absolutely not affected by aggregation of the substances involved in the process. In addition, in the mentioned case, the specific aggregation of the agents due to binding of several agents to one molecule of BSA may modify the fluorescent signal due to, for example, fluorescence quenching. However, if aggregation is insignificant and BSA is added in sufficient amounts, the fluorescence quenching only slightly affects the signal. In the mentioned cases, the object that produces the output action may be a detectible label (fluorescent, magnetic, exhibiting surface plasmon resonance properties), which directly produces the output action in the form of detectible signal; besides, it may be, for example, an enzyme label that produces fluorescent molecules from non-fluorescent, etc. Then the output action should be produced by said object rather than result from interaction with the agent. Besides, in the cases when said object changes the output action produced by the blockable label, said object may be, for example, a fluorescence quencher of the blockable label or an inhibitor of an enzyme blockable label. At this, the output action should be defined by the blockable object rather than by the agent (unlike the case of determining the aggregation of agents), and the requirement for said object to be different from the agent (or its analog) means that a change of the output action can only be achieved through the interaction of said object with the blockable label irrespective to interaction of the agents with each other. Besides, said analog of the agent means an analog constructed similarly to the agent, but having different linking receptors, other blockable labels or other corresponding blocking substances. Besides, in another embodiment of the invention, when the blockable label binds to a surface of a solid phase, the agents can produce the output action themselves, for example, due to the fact that they can be registered (if, for example, they are colored latex particles or gold particles, or magnetic particles, or are associated with other labels, for example, enzyme ones) or can be, for example, cytotoxic upon delivery to a cell; and the specific binding of the agents with the solid phase (including with the surface of a cell) is not, as such, their aggregation. Although a large number of particles bind to a "single" solid phase, such interaction should be attributed to specific binding of the agents with the objects that are not analogous to them, rather than to aggregation of the agents with each other in the sense of aggregation of analogous substances. In other embodiment of the invention, said output action is produced by said blockable label of said agent depending on a direct and indirect interaction of said blockable label with other molecules or particles, or surfaces of solid phase, not including those located on other agents, which are identical or similar to said agent, i.e., which have other blockable labels and/or linking receptors, and/or corresponding blocking substances.

Besides, the output action may consist in producing, modification, removing from a medium (in the sense of transformation to other molecules or sorption) of some molecules. For example, if the blockable label is enzyme, then while interaction with its substrate, the blockable label may modify the substrate, cleave it, etc. Besides, for example, the blockable label may be a substrate, interacting with which an enzyme produces or displaces from a medium other molecules. For example, the blockable label may be a single stranded oligonucleotide. Then in the blocked state, the access to the blockable label of DNA polymerase (e.g., immobilized on a nanoparticle) is difficult or impossible. In the case of unblocking, it is possible to conduct a polymerase chain reaction (PCR) and produce large amounts of this oligonucleotide. In another embodiment, in the blocked state a DNAse cannot approach to an oligonucleotide blockable label and cannot cleave its part, which can be afterwards amplified by means of PCR.

In one embodiment of the invention, due to employment for the spatial or spatial-electrostatic blocking of the blocking substance and the agent, which exceed in size the receptors used in the system, as well as due to spatial separation of the linking receptor and the blockable label of the agent it is possible to use as the blockable labels, inter alia, enzymes capable of degrading the receptors, via which the blocking substance binds with the agent. Through the use of an agent that carries, for example, a protein receptor and a blockable label, which is a protease, the protease virtually has no opportunity to degrade a receptor on other agents due to limited diffusion and limited degrees of freedom, i.e. no possibility of correct spatial orientation of the receptor with respect to the protease for its degradation. In this case, the protease may be functional with respect to a low-molecular substrate that has fast diffusion and is unlimited in all degrees of freedom. The same is applicable to other enzymes: nucleases, glycosidase, etc. For example, in one embodiment of the invention, concanavalin A, which binds terminal residues of mannose and glucose, and enteropeptidase are immobilized on the agent. Then the blocking substance may be a cross-linked horseradish peroxidase, which is well bound by concanavalin A and eluted from this bond by glucose. As a substrate, trypsinogen (including that immobilized on nanoparticles) can be used. Then in the case of blocking the interaction of enteropeptidase with trypsinogen due to binding the agent with the blocking substance (at low concentration of glucose), trypsinogen is not activated into trypsin. With increasing concentrations of glucose, enteropeptidase is made unblocked and transforms trypsinogen into trypsin. If the solution contains procarboxypeptidase B, then it is transformed by trypsin into an active carboxypeptidase B enzyme, which, combined with trypsin, can enzymatically transform proinsulin into active insulin (W. Kemmler, J.D. Peterson, D.F. Steiner, Studies on the conversion of proinsulin to insulin. I. Conversion in vitro with trypsin and carboxypeptidase B, J. Biol. Chem. 246 (1971) 6786-6791). This way one can achieve production of insulin from proinsulin when concentration of glucose in a medium increases, and this may be a very efficient way for treatment of diabetes.

It should be noted that, generally, when unblocked and present on the agent, the blockable label should produce an output action. However, it should be mentioned that the other cases, namely: not producing any action by the blockable label, when it is unblocked and present on the agent, and producing the action by the blocked blockable label or by the label not bound with the agent, can be also considered as the value of output of logic element complex equaled to 1 (TRUE).

The present invention may also be used for diagnostic and/or therapeutic purposes, including targeted delivery of various agents to the cells. These may be cells grown *in vitro* in culture or cells of a living organism. The method can be applied, for example, as follows. If, for illustrative purposes, it is required to deliver magnetic nanoparticles (MP) to tumor cells, these MP can be a basis of the agent. They can be conjugated with two receptors: the first, which is the blockable label, represents an antitumor antibody having specificity to oncomarkers on the cell membrane, e.g., commercially available trastuzumab antibody; the other receptor (linking receptor 1) is a receptor to a nontoxic low-molecular compound (input) such as concanavalin A, which is a receptor to glucose. As the blocking substance, a nanoparticle may be used, which is a cross-linked protein recognizable by concanavalin A. This may be a protein of non-animal origin, such as horseradish peroxidase, but a protein of a specie, for which the therapeutic or diagnostic agent is created, is preferable. A protein obtained from blood of the particular organism, for which the agent is created, is substantially advantageous. This eliminates immunogenicity of the administered agent and minimizes opsonization. Besides, in the case of highly sialated blocking substance reliable masking of the agent from organs of the reticulo-endothelial system, which removes foreign particles from the bloodstream, becomes possible.

When combining MP conjugated with said receptors with the blocking substance, an assembled agent of logic element complex is obtained which can be separated from the blocking substance unbound to MP conjugates. However, the efficiency of the agent delivery to a tumor may be increased if the unbound component is not removed. Upon introducing of a free blocking substance into the bloodstream (the introducing of the blocking substance is possible even some time earlier than that of the logic element complex), the filter systems of the organism (e.g., the Kupffer cells) becomes busy filtering the free blocking substance, and when the logic element complex is introduced, it is cleared from the bloodstream more slowly, thus increasing the time for the blockable label to recognize its target.

Upon introducing the logic element complex in the human body, the blockable label is not immediately exposed on the surface of the agent. This allows avoiding opsonization of the foreign blockable label on the agent and permits masking the surface of the particle by protein of the organism, which also leads to a decrease of opsonization. After uniform distribution of the logic element complex over the bloodstream and throughout the organism, it is necessary to raise the level of the input signal, which in this example is glucose. This can be done either by direct injection of glucose into the bloodstream or orally, i.e. allowing the patient to eat glucose if permitted by the pharmacokinetic parameters of the logic element complex, i.e., if it circulates long enough for uptake of glucose in the stomach. The higher level of glucose, the faster the dissociation of the blocking substance from MP and uncovering of the blockable label, which in this case is antibody capable of recognition of a tumor marker on a cancer cell and thereby deliver MP to the target cell. Under this variant of delivery of the particle, it is possible to distribute the particles uniformly in the bloodstream as long as they are protected from opsonization by the organism's protein, and only then "turn on" their recognition component.

Besides, an option is possible that the input signal can be selected so that it does not require to be specially introduced into the bloodstream but its concentration is increased in the area where the agent should be delivered to. For example, in the areas of inflammation, the concentration of chemoattractants that attract neutrophils and monocytes is increased; the pH is lower in tumors, etc.

Besides, the logic element complex can be fabricated as a multi-layered structure. For example, the first "layer" immobilized on the magnetic particle may consist of the blockable label A and the linking receptor 1A bound with the blocking substance A that blocks the blockable label A. Such a nanoparticle may be the basis for the second layer. For example, on said blocking substance A, a blockable label B and a linking receptor 1B bound with the blockable substance B, which blocks the blockable label B, are immobilized, etc. (the same is possible when using the linking receptor 2 for binding of the blockable label with the agent, which is MP). This variant can be used, for example, for sequential delivery of the particle to different "nested" targets - for example, first to a membrane of a specific cell, then providing escape from lysosomes, then to the nucleus. "Activation" of the corresponding blockable labels may be facilitated by different biochemical environment in the respective areas (blood flow, lysosome, cytoplasm, etc.).

Due to the fact that the logical element complex not only can recognize a single input but also can, for example, "enable/disable" the blockable labels if certain input signals are present, as well as in the absence of other certain input signals, it is possible to create a substance that "enables" its action only under a particular biochemical "profile" of the environment (i.e., under a certain mutual proportion of different molecules in the medium). This can be useful for diagnosis and treatment of complex diseases (such as cancer) for which single highly-specific identifying markers are so far unknown but groups of markers are known, which may be used for more accurate diagnosticsif the lack of some markers in the group and simultaneous presence of other markers in the group is registered. In this case, a substance capable of simultaneous detecting the presence of certain molecules and the absence of other molecules has an obvious advantage over the current techniques for targeted delivery, which generally operate in terms of the presence of a disease marker.

Said blockable label may be not only a receptor for targeted delivery, but also, for example, an enzyme whose interaction with its substrate is blocked by a blocking substance, or a fluorescent label which is blocked from interaction with a fluorescence quencher by a blocking substance, or alternatively, the blockable label may be a fluorescence quencher whose interaction with a fluorophore is sterically blocked by a blocking substance.

For example, if the blockable label is an enzyme, e.g., ricin (or ricin A chain), then its cytotoxicity defined by cleavage of glycosidic bonds in ribosomes, which blocks synthesis of protein, may be spatially blocked by a blocking substance, which does not sterically permit a large ribosome to approach the ricin on the agent. If the blocking substance dissociates from the agent, the ricin "turns on" and kills the target cell. Besides, when using ricin consisting of an enzyme part (A chain) and lectin part (B chain), which is responsible for penetration of ricin into cell cytosol and binds to residues of galactose and N-acetylgalactosamine, it is possible to use the ricin B chain as the linking receptor and a particle that carries the residues of galactose and N-acetylgalactosamine - as the blocking component.

As said blocking substance, one of the input signals (e.g., an antibody against the linking receptor 1) or a molecule, particle, or a surface carrying certain receptors that directly or indirectly bind to the linking receptor 1 may be used, one or more input signals affecting this bond.

Besides, the role of the blocking substance can also be performed by any other particle or molecule capable of binding to the linking receptor 1 of the agent by means of an input signal or on its own (in the latter case, the dependence of the bond between the blocking substance and the agent upon the input signal is governed by the ability of the input signal to destroy this bond. For example, when using a molecular input signal, the blocking substance may be created by cross-linking of a receptor to the input signal or cross-linking of the molecules of the input signal (or its analog), as well as by cross-linking of the carrier molecule that carries the molecule of the input signal (in the case of small-molecule input). Moreover, for example, if the input signal is a carbohydrate, e.g., glucose or mannose, the carrier molecule may naturally bear this input signal, e.g., in the case of glycoprotein of horseradish peroxidase. Besides, the input signal may be immobilized on the carrier molecule artificially, for example, by chemical conjugation, e.g., the same way as it is commonly done while immobilization of haptens on carrier protein for immunization of animals and generation of antibodies against this hapten. Besides, the input signal or its receptor may be conjugated with a particle formed by cross-linking of another molecule. Cross-linking may be implemented by various known methods, for example, by homobifunctional cross-linkers (gluteraldehyde, etc.) or heterobifunctional ones. Besides, functional groups may be formed on such artificial protein particle for convenient and rapid conjugation of haptens, ligands or receptors. Using of such a particle is convenient because by adjustment of concentrations of the cross-linkable molecule and cross-linking agent it is possible to get particles of different sizes to optimize their properties for steric blocking of the agent surface. The dispersion in size of the obtained blocking particles can be reduced by selecting fractions with the desired size using the gel filtration chromatographic separation of the cross-linked molecules. At this, it is possible to select the size of the blocking particles for optimal blocking of the blockable labels. Besides, using different fractions of the particles after the gel filtration of the cross-linked molecules, better steric blocking of the blockable labels can be realized if the fraction with larger particles is added first followed by addition of smaller particles.

In addition, if using the agents having many linking receptors 1, it is possible to make the binding of the blocking substance to the agent one-point or multipoint. The case of multipoint binding is favorably distinguished because it is possible to use low-affinity receptors to the input signal or receptors that feature high speed of dissociation of the complex with the input signal. In this case, the sensitivity of the logic element and the speed of its operation are higher than those when using of high-affinity receptors if the input signal is added to the already assembled "particle-blocking substance" complex. Due to rapid dissociation of the "receptor-epitope of the blocking substance" pairs, the displacement of the blocking agents from interaction upon addition of lower concentrations of free detectable input signal is more likely. Nevertheless, because of the multipoint binding the bond between the particle and the blocking substance is strong in the absence of the free input signal. This case is advantageous if the response time of the logic element is critical.

It should be noted that the blocking substance may interact with the agent not only through interaction via the linking receptor 1. For example, by using a second bond between the agent and the blocking substance, a repeatedly reversible blocking of the blockable label on the agent can be realized. If said second bond (covalent or noncovalent) is realized using a long linker, the blocking substance is made unbound from the linking receptor 1 on the agent by, for example, displacement with a free molecular input signal, and moves away to a distance defined by the length of said linker, thereby unlocking the blockable label for producing the output action. When concentration of the input signal in the sample decreases, the molecules of the input signal dissociate from the linking receptor 1 on the agent, which again becomes available for binding with the blockable substance so that the blockable label becomes blocked, thereby finishing producing the output action. This linker may be different molecules, however, hydrophilic polymers (polyethylene glycol, dextran, etc.) are preferable because they do not prevent but even stimulate a removal of the blocking substance from the agent as long as they are not bound through the linking receptor 1 on the agent.

The ability of the blocking substance to bind with the agent directly or indirectly via the linking receptor 1 on the agent assumes a wide variety of options for blocking the blockable label on the agent with the blocking substance. For example, the blocking substance may bind to the linking receptor 1 on the agent not directly, but through a set of molecules interacting with each other; for example, if murine antibody against molecular input signal is used as the linking receptor 1, the biotinylated input signal (or biotinylated analog of the input signal, to which said antibody has less affinity so that the input signal, upon addition, easily displaces said biotinylated analog) may be added first followed by addition of the blocking substance that carries (strept)avidin. This may for instance allow the use of only one type of blocking substances for many input signals.

Besides, a multilayer blocking can be used, including, for example, the above case with the streptavidin blocking substance where the second blocking substance that carries rat anti-streptavidin antibody, and the third blocking substance that carries rabbit anti-rat antibodies, etc., can be used. It is clear that the blocking may be substantially enhanced when using of such multilayer blocking.

In one embodiment of the invention, nonspecific binding of the blocking substance with the agent does not worsen the sensitivity of the logic element complex to input signal. In the known methods for detection of different molecules, for example, in ELISA, non-specific binding of an enzyme label (that interacts with the immunosandwich) with solid phase of the plate causes an increase in all signals (for all samples), which worsens the detection limit of the method. In the present invention, for example, in the embodiment where the blockable label for producing an output action binds to a molecule immobilized on a solid phase (e.g., on an immunochromatographic test strip), non-specific binding of the blocking substance with the agent, on the contrary, leads to a decrease of the signal rather than to its increase, and does not reduce the sensitivity to input signal. In one embodiment of the invention, non-specific binding of agents with a solid phase via a blocking substance that is itself nonspecifically bound with the agent does not occur due to more probable nonspecific binding with the solid phase of the free blocking substance that is usually present in the mixture in substantially greater amounts than the number of the agents. Besides, by choosing the blocking substance that features minimal nonspecific binding to the solid phase (but not necessarily to the agent), it is possible to reduce harmful effects of nonspecific binding in the system virtually to the absolute minimum. For this purpose, in one of the embodiments of the invention, it is advantageous to use a blocking substance created based on highly hydrophilic non-charged compounds which usually exhibit minimal nonspecific binding. This may be, for example, particles coated by polyethylene glycol molecules or created with substantial amounts of carbohydrates, e.g., on the basis of dextran or glycoproteins. Besides, in the above examples, the nonspecific binding by means of the receptors on the blocking substance, which directly or indirectly recognize the receptor 1 on the agent, may not worsen the detection limit. As it is mentioned above, both such nonspecific binding with the agent and nonspecific binding of the free blocking substance with the solid phase do not increase the signal. Thus, in one of the embodiments, the system may be virtually completely independent on nonspecific binding of the blocking substance with the agent, if their specific binding depends on the input signal.

The sensitivity of the system to input signal (i.e. the threshold strength of the signal, when its value changes from zero to one) can be shifted to the desired range of, for example, concentrations (in the case of a molecular input signal) by adjusting the density of, usually, the linking receptors (1 or 2) on the agent, though a variant of adjusting the density of the blockable label on the agent may also be realized. For example, the following variant is possible. In the case of high demands to sensitivity of the logic element complex (i.e., the requirement of destruction or formation of a bond between the blocking substance and the agent), sparse positioning of the linking receptors 1 on the agent is preferable, but with the possibility for the blocking substance to block the blockable labels bound with the agent. Then upon addition of a small amount of molecules of the input signal and inhibition of at least one linking receptor 1 on the agent, the respective blocking substance cannot bind to the agent at this site, so the blockable labels at this site remain unblocked and may bind with a complementary ligand, e.g., immobilized on an immunochromatographic test strip. In the case of dense positioning of the linking receptors 1 on the agent, more molecules of the input signal are required to prevent binding of the blocking substance with the agent at a definite site of the agent due to, firstly, higher concentration of the linking receptors, and secondly, because of possible multipoint binding of the blocking substance and the agent.

Besides, the density of blockable labels on the agent or the number of linking receptors 2 capable of binding the blockable labels determines the complexity of the complete blocking of the blockable labels bound with the agent, and that also affects the sensitivity of the logic element complex with respect to the input signal.

The longer the distance of spatial separation of the linking receptor 1 and linking receptor 2 (or the blockable label) on the agent, the more difficult to block spatially or spatially-electrostatically the blockable label. At this, for example, when a molecular input signal itself serves as the blocking substance, the method is limited to very large input molecules (signals). However, if using of an additional blocking substance to block the blockable label it is possible to block the blockable labels that are substantially distant from the linking receptors, and this distance may be of the order of the size of the blocking substance. Taking into account that the blocking substance may be substantially larger than any molecular receptor (up to several tens of microns or even larger), the present invention is no way limited by the size of molecules of the input signals or by the distance of the spatial separation of the linking receptor 1 and linking receptor 2 (or the blockable label).

A change of the output action depends on the fact of binding or not binding of the blocking substance with the agent. At this, the input signal may affect the formation or destruction of the bond between the blocking substance and the agent in various ways. For example, binding of the blocking substance with the linking receptor on the agent may occur, inter alia, in the following two ways. First, the mentioned binding may be realized due to recognition by the linking receptor 1 of the input signal molecules on the blocking substance or due to recognition of the input signal molecules on another molecule, with which the blocking substance binds directly or indirectly. In this case, the input signal competes for binding with said linking receptor 1, so the amount of the blocking substance bound with the agent depends upon the amount of the input signal. The second variant is realized in a different way. The blocking substance binds with the linking receptor 1 on the agent due to recognition of the linking receptor 1 on the agent by the receptor on the blocking substance, or due to direct or indirect binding of the blocking substance with the receptor bound to the linking receptor 1. At this, binding of said receptor on the blocking substance with the linking receptor 1 is hindered or impossible in the case of direct or indirect binding of the linking receptor 1 with molecules of the input signal.

Spatial or spatial-electrostatic blocking of the blockable label means inhibition of direct or indirect interaction of the blockable label with certain other molecules due to steric and/or electrostatic factors. That means that said certain molecules cannot approach the blockable label because of the presence of the blocking substance near the blockable label, thus producing the "blocking". The inability to approach may be due to the volume of the blocking substance and also to its strong charge. For example, when the charges of the blocking substance and said molecules are of the same sign, said molecules are repulsed from the blocking substance and do not approach the blockable label. In the case of different signs of the charges, said molecules may be attracted to the blocking substance, thereby their interaction with the blockable label competes with attraction to the blocking substance, and hence an effective "blocking" is realized.

Besides, the blocking may be enhanced as follows: if the blocking substance has, inter alia, weak affinity to the blockable label, then, compared to the case when no affinity is available, substantially stronger blocking may be achieved in the absence of the input signal and insignificantly changed residual blocking in the presence of the input signal and said molecules that interact with the blockable label.

Since all reversible biochemical reactions of interaction imply the presence of both bound and free molecules, all the above used notions such as "blocking", "inability to approach ", etc., are used here in statistical sense, i.e. for example, the term "blocking" can be used even if not all blockable labels are "blocked", it is only important that in the case of blocking there are more "blocked labels" than the "not blocked".

Besides, for the ease of explanation the blockable label mentioned in the examples given in this description is believed to be the same for said agents. However, this is not necessary, i.e., the blockable labels may be different (dissimilar). In that case one should understand that the notion of the value of a logical function should be interpreted differently, for example, it is equal to 1 (true) if at least one blockable label produces a corresponding output action. For example, it is convenient to use various oligonucleotides as such a set of dissimilar blockable labels because of a large variety of specifically interacting pairs. In this case one may receive not only the integral information about the value of the function of the logic element complex but also the detailed information about the status of each agent within the logic element complex.

In one embodiment of the invention, an agent is selected, which represents a nanoparticle or a microparticle, or a surface of a solid phase and carries at least a linking receptor 1 and a blockable label, said agent being involved directly or indirectly in producing an output action; a blocking substance is also used that is capable to bind directly or indirectly with the linking receptor 1 on the agent. Then the blocking substance may bind to the linking receptor 1 due to the fact that it is composed of molecules of the input signal (or its analogue) or carries these (or similar) molecules. At this, localization of the linking receptor 1 on the agent is beneficial because in this case a small amount of the input signal is sufficient to prevent blocking of the blockable labels with the blocking substance unlike the case when, for example, the molecules of the input signal are positioned on the agent while their receptors are on the blocking substance because in the latter case the blocking substance and the receptors to the input signal on the blocking substance should be present in significantly larger amounts than that of the molecules of the input signal on the agent.

Besides, the blocking substance may bind to the linking receptor 1 in various indirect ways, for example, it may interact with a chain of molecules or particles, one of which carries or consists of molecules of the input signal; or the blocking substance may carry or consist of receptors to the molecules of the input signal, then the molecules of the input signal bind to the linking receptor 1 (also being the receptor to the molecules of the input signal) on the agent, and after that said blocking substance binds to the bound molecules of the input signal. Besides, various other molecules, e.g., biotin-streptavidin, etc., may participate in the chain of bonds between the agent and the blocking substance.

Besides, in one of the embodiments of the invention, an agent is used that carries the linking receptor 1 and the blockable label. This approach is advantageous because, firstly, such agent-carrier carries simultaneously a large number of the linking receptors 1 and of the blockable labels that produce the output action.

Secondly, the use of such an agent rather than direct binding of the linking receptor 1 with the blockable label allows spatial separation of the linking receptor 1 and the blockable label; this considerably complicates blocking, but may help to immobilize more blockable labels in active state. Namely, due to the fact that said linking receptors 1 and blockable labels are bound indirectly, they do not block or disrupt activity of each other. That is, for example, under conjugation of a large number of fluorophore with antibody (i.e., if the blockable label, which is a fluorophore, is directly conjugated with the linking receptor 1, which is an antibody) insolubilization may occur because most commonly used fluorophores are poorly soluble in water. Furthermore, the fluorophore usually binds chemically and thus randomly, so a part of the fluorophore may bind to the recognition site of the antibody, which does not allow it to recognize the ligand, which is an input, and, in addition, when there is large amount of the fluorophore on the antibody, the cross quenching of the fluorophore occurs.

Third, said spatial separation of said linking receptor 1 and the blockable label and the use of the blocking substance (rather than the molecules of the input signal) for spatial blocking of the blockable labels permit employment as the linking receptor 1 receptor and the blockable label of molecules or substances of almost any size - up to several micrometers or more. Besides, by using large agents that are significantly larger than the size of said molecules, a fundamentally different (as compared with the case when the linking receptor 1 is directly bound with the blockable label) spatial or spatial-electrostatic blocking of the blockable labels becomes possible, namely, the layered blocking, including the multi-layer one with using of multiple types of the blocking substances. In this case, the efficiency of blocking the blockable labels may be made almost independent of their amount on the agent, i.e. the efficiency of the blocking only depends on the amount of the linking receptors 1 on the agent. When minimal amount of the linking receptors 1 sufficient to form a "complete blocking layer" is available, all blockable labels on the agent become blocked. In the case of direct binding (e.g., conjugation) of the linking receptors 1 and the blockable label the efficiency of the blocking substantially depends on the ratio between these two entities. Under larger amount of the linking receptors 1, the efficiency of blocking is higher but the output action from the blockable labels is weaker, which imposes considerable limitations on applicability of the invention.

Fourth, said agent allows its using as a solid phase, i.e. provides easy separation (e.g., if the agent is a magnetic nanoparticle or microparticle) and other benefits of using nanoparticles or microparticles as a solid phase.

Besides, in one of the embodiments, when using an agent that carries at least the linking receptor 1 and the blockable label that participates directly or indirectly in producing the output action, spatial or spatial-electrostatic blocking of said blockable label, which causes a change in said output action, may occur due to molecules of the input signal that bind to the linking receptor 1. In this case, the input signal itself represents the blocking label. Then aforementioned "layered" blocking that provides all the above mentioned advantages is also possible.

Besides, in one of the embodiments of the invention, an agent is used, which has at least the linking receptor 1 and the blockable label that participates directly or indirectly in producing the output action. At this, the agent is created so that said output action is performed to a higher extent when the blocking substance and the agent do not bind with each other, and is produced to a lesser extent in the case of their binding. The inverse variant is also possible.

Besides, in another embodiment, any input signal, which affects binding of the blocking substance and the agent, does not affect binding of the blockable label and the agent, and vice versa, any input signal, which affects binding of the blockable label and the agent, does not affect binding of the blocking substance and the agent.

Along with simple logic element complexes, creation of substantially more sophisticated logic element complexes and cascading (combining) of these logic element complexes into logic circuits are possible in frames of the present invention.

An output action of one logic element complex (CLE) may be an input for another logic element complex, i.e., the signal from one agent may be transmitted to another agent. For example, if the output action of the first CLE is production of a molecule, then, as described above, the increased concentration of such molecules may, for instance, break the bond between the agent and the blocking substance of the second CLE. This way a logic circuit may be created, the output of one CLE being the input of other CLEs.

In addition, the output action of a CLE may be, for example, production of, inter alia, molecules identical or similar to the input signal of this CLE, and thereby a CLE can be realized that corresponds to a non-linear CLE, an amplifier of the input signal.

Besides, the output action of a CLE may be, for example, production of, inter alia, molecules that inhibit, neutralize or transform the input signal of this CLE, and thereby a CLE can be realized that corresponds to a non-linear CLE, a quencher of the input signal.

Besides, said circuit and single CLEs may be made substantially more sophisticated by several factors:
1) each CLE may have many different outputs in the sense that the "activated" state may be expressed by an output action consisting in production of several different molecules or other effects. At this, each produced molecule or effect may serve as an input to another or the same CLE.
2) the same CLE may "activate" the production of different molecules (or other output actions) under quantitatively different unblocking of the blockable label. For example, if the product of said molecules is due to enzyme blockable labels, and the process of production of said molecules represents degradation by said enzymes of their substrates. At this, for different enzymes (e.g., glucosidase and DNAse), sizes of the substrates may range substantially. This way, a variant of multivalued logic may be realized by converting the quantitative degree of blocking of the blockable label in qualitatively diverse output actions, for example, "there is no modification of substrates", "modification of only small substrate", "modification of small and large substrates."

Operation of the logic element complex is illustrated by drawings in Figs. 1-3.

### List of drawings:

Fig. 1/3. Logic element complex for implementation of YES logic function.
Fig. 2/3. Logic element complex for implementation of NOT logic function.
Fig. 3/3. Logic element complex for implementation of (YES A)&(YES B)&(YES C)&(NOT i)&(NOT ii)&(NOT iii)) logic function of variables A,B,C, I, ii, iii.

### Examples

The following examples are given to illustrate the invention and do not limit its application.

Embodiments for implementation of logic functions using the logic element complexes (CLE) are broad-ranging. Various examples of implementation of such functions are given below.

Let a portion of the input signals identifies binding of the blockable label with the agent, and at least another portion identifies binding of the blocking substance with the agent. Then it is possible to implement a set of functions YES, NOT, AND, OR, i.e. a basis for expression of any logical function by the following variants.

Let us consider two boundary variants for implementation of logic functions using the invention: the first one, in which all input signals destroy some bond, and the second one, in which all input signals mediate formation of some bond.

### Example 1. In this example, the input signals that destroy some bond are used

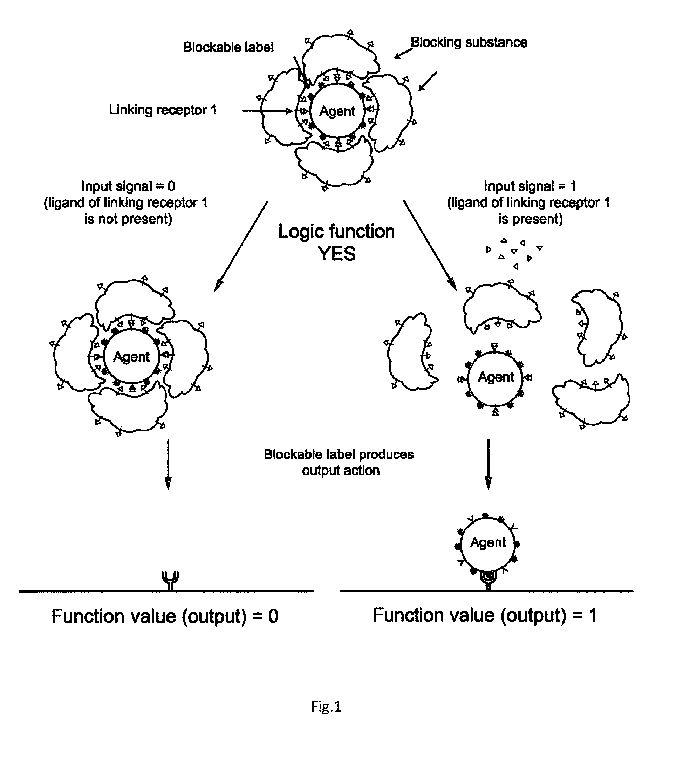

The function "YES" is implemented by a CLE (Fig. 1), wherein the blocking substance is bound with the linking receptor 1 of the agent that carries the linking receptor 1 and the blockable label, the bond between the blocking substance and the agent depends on the input signal. If the input signal is capable to break said bond, then the "YES" function is realized by means of said CLE. Indeed, if the input signal destroys said bond, the blockable label on the agent is unblocked, and the output action is performed.

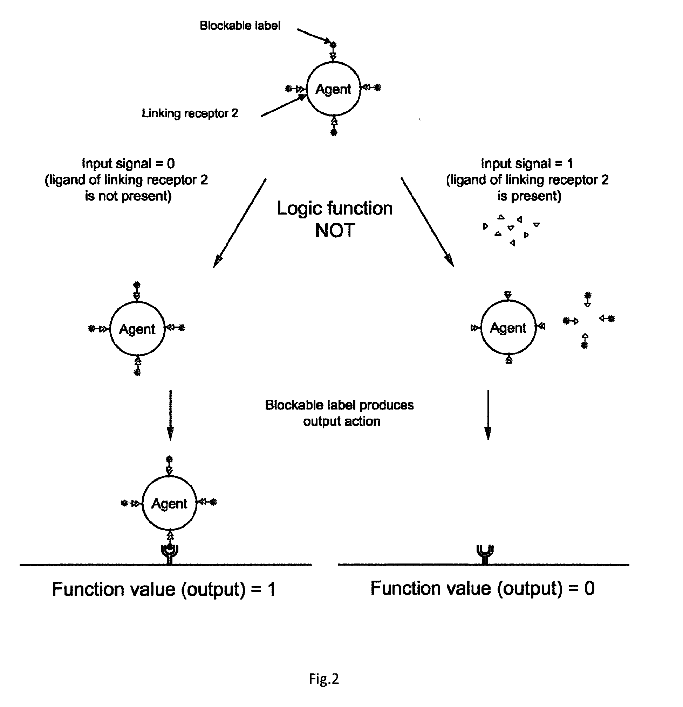

The "NOT" function is realized by a CLE (Fig. 2), in which the agent that carries the linking receptor 2 is bound with the blockable label via the linking receptor 2 depending on the input signal. If the input signal breaks said bond, this CLE implements the logic function "NOT". For example, if the input signal destroys said bond, the blockable label dissociates from the agent, so that no blockable label is available on the agent to produce an output action (e.g., bind the agent with the surface of solid phase).

The function of conjunction of variables [(YES A) AND (YES B)] is implemented by a CLE, in which the agent carries simultaneously a set of the linking receptors 1 for all input signals, and the blocking substance is bound or is capable of binding with all the mentioned linking receptors 1. At this, if the input signals are meant to destroy the corresponding bonds of the linking receptor 1 with the blocking substance, then in the absence of at least one input signal binding of the agent and the blocking substance cannot be broken, and the blockable label becomes blocked.

The function of disjunction of variables [(YES A) OR (YES B)] is implemented by a CLE, comprising various agents for each input signal, all the agents carrying the same blockable label and each agent carrying the linking receptor 1 that corresponds to only one input signal; each linking receptor 1 is bound with at least one blocking substance or a set of blocking substances such as at least one blocking substance can bind to each linking receptor 1. In this case, if the input signals break the corresponding bonds between the linking receptors 1 and the blocking substances, then only in the absence of all input signals the agents remain bound with the corresponding blocking substances and the blockable labels on all agents remain blocked. If even one input signal is present, the blockable label on the appropriate agent becomes unblocked, and the output action is produced.

The function of conjunction of negations of variables [(NOT A) AND (NOT B)] is realized by a CLE, in which the blockable label is bound with the linking receptor 2 of the agent through a set of consecutive bonds, including the bonds breakable by all input signals, where upon destruction of the respective bond by any input signal, the blockable label dissociates from the agent. These set of consecutive bonds is realized as follows. The linking receptor 2 of the agent binds to molecule 1 so that their bond is destroyed by the input signal 1, said molecule 1 is bound with molecule 2 that carries the blockable label, the bond between the molecule 1 and molecule 2 is destroyed by the input signal 2. In the case of a greater number of the input signals of the CLE, the used set of the molecules 1,2, ..., n increases, including the molecules, the bonds of which are destroyed by the respective input signals. At this, the n molecule carries, inter alia, the blockable label.

The function of disjunction of negations of variables [(NOT A) OR (NOT B)] is implemented by a CLE, in which the blockable labels are bound with the linking receptors 2 of the agent by a set of parallel bonds destroyable by the input signals, where upon destruction by the input signal of the respective bond, the respective blockable label dissociates from the agent. Such a set of parallel bonds is implemented as follows. The linking receptor 2 of the agent is bound with the molecule 1 that carries the blockable label so that the bond between them is destroyed by the input signal 1, and, in addition, the linking receptor 2 of the agent is bound with the molecule 2 that carries the blockable label so that the bond between them is destroyed by the input signal 2, etc.

Another realization of the function of disjunction of negations of variables [(NOT A) OR (NOT B)] is realized by a CLE, in which the identical blockable labels are bound with the linking receptors 2 of a set of different agents, uniquely correspondent to each of the input signals, through the bonds destroyable by the input signals, and upon destruction by the input signal of the respective bond, the blockable label dissociates from the respective agent. Such set of bonds is implemented as follows. The linking receptor 2 of the agent 1 is bound with the molecule 1 that carries the blockable label so that the bond between them is destroyed by the input signal 1; the linking receptor 2 of the agent 2 is bound with the molecule 2 that carries the blockable label so that the bond between them is destroyed by the input signal 2, etc.

### Example 2. In this example, the input signals that mediate binding are used

The "YES" function is implemented by a CLE, wherein to the agent, which carries the linking receptor 2 but does not carry the active blockable label, the blockable label binds via the input signal that binds to the linking receptor 2. Then the agent gets on its surface the active blockable label capable of producing the output action.

The "NOT" function is implemented by a CLE, wherein the agent that carries the linking receptor 1 and the active blockable label binds with the blocking substance via the input signal that binds to the linking receptor 1; as a result, the blockable label becomes spatially or spatially-electrostatically blocked and cannot produce the output action.

The function of conjunction of negations of variables [(NOT A) AND (NOT B)] is realized by a CLE, in which the agent carries a set of the linking receptors 1, corresponding to the input signals, and the active blockable label. Besides, the CLE includes a set of the blocking substances such that for each input signal there is the blocking substance capable of binding with the corresponding linking receptor 1 of the agent via said input signal to block the blockable label. Then, if at least one of the input signals is present, the blocking substance binds to the agent and spatially or spatially-electrostatically blocks the blockable label so that it cannot produce the output action.

The function of disjunction of negations of variables [(NOT A) OR (NOT B)] is implemented by a CLE, which includes a set of the agents that carry identical active blockable labels and each such agent carries the linking receptor 1 that uniquely corresponds to one of the input signals. Importantly, for each input signal there is a corresponding agent within the CLE. Besides, the CLE includes a set of the blocking substances such that for each input signal there is the blocking substance capable of binding with the linking receptor 1 of the corresponding agent via said input signal to block the blockable label on this agent. In this case, to lock all the blockable labels on all agents, the presence of all input signals is obligatory.

The function of conjunction of variables [(YES A) AND (YES B)] is implemented by a CLE, wherein the blockable label binds with the linking receptor 2 of the agent through a set of consecutive bonds, include the bonds mediated by all the input signals, and only if all the input signals are present, the blockable label is capable to bind with the agent. Such a set of consecutive bonds is implemented as follows. The linking receptor 2 of the agent binds with a molecule 1 so that the bond between them is mediated by the input signal 1, a molecule 2 that carries the blockable label binds with said molecule 1 via the input signal 2. In the case of a larger number of the input signals, the used set of molecules 1,2, ..., n increases by including the molecules, the bonds of which with the previous ones are mediated by the corresponding input signals. At this, the molecule n carries, inter alia, the blockable label.

The function of disjunction of variables [(YES A) OR (YES B)] is implemented by a CLE, in which the blockable labels are bound with the linking receptors 2 of the agent by a set of parallel bonds mediated by the input signals, wherein in the absence of any of the input signals the corresponding blockable label cannot bind to the agent. Such a set of the parallel bonds is realized as follows. The linking receptor 2 of the agent is bound with a molecule 1, which carries the blockable label, so that the bond between them is mediated by the input signal 1, besides, the linking receptor 2 of the agent is bound with a molecule 2, which carries the blockable label, so that the bond between them is mediated by the input signal 2, etc.

Another implementation of the function of disjunction of negations of variables [(NOT A) OR (NOT B)] is realized by a CLE, in which the identical blockable labels are bound with the linking receptors 2 of a set of different agents, uniquely correspondent to each of the input signals, through the input signals, and in the absence of some input signal the blockable label cannot bind with the corresponding agent. Such a set of bonds is implemented as follows. The linking receptor 2 of the agent 1 is bound with a molecule 1, which carries the blockable label, so that the bond between them is mediated by the input signal 1; the linking receptor 2 of the agent 2 is bound with a molecule 2, which carries the blockable label, so that the bond between them is mediated by the input signal 2, etc.

Thus, the abovementioned embodiments enable implementation of the set of functions YES, NOT, OR, AND, NOT OR (conjunction of negations), NOT AND (disjunction of negations)" both in the case of the input signals that destroy bonds (Example 1) and in the case of the input signals that mediate bonds (Example 2). These sets allow expression of any logic function through the functions of each set.

Composite functions based on a complete functional set of conjunction and disjunction of the values of the variables and their negations in the case of the input signals that break the relevant bonds.

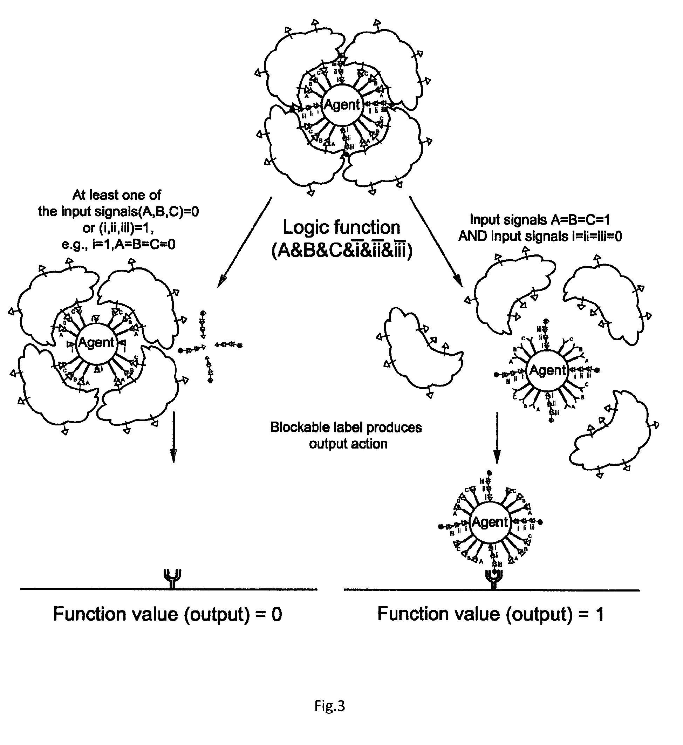

**Example 3.** The CLE that implements a logic function, which is a combination of the logic functions YES, NOT, AND (i.e., conjunction of variables and their negations), is realized as follows (Fig. 3). The CLE constists, inter alia, of the agent, with which the blockable label is bound by bonds that are destroyed by the input signals that correspond to the negated variables in the logic function. Besides, the agent carries a set of the linking receptors 1, which bind with the blocking substance of the CLE, wherein for each input signal that is comprised in said composite logic function as a value of variable (rather than its negation), there is at least one linking receptor 1, the bond of which with the blocking substance is destroyed by said input signal, and for each linking receptor 1 there is an input signal that is comprised into said composite logic function as a value of variable (rather than its negation), which breaks the bond between said linking receptor 1 with the blocking substance.

**Example 4.** Using the CLE shown in Example 3 for implementation of a combination of the logic functions YES, NOT and AND (i.e, the conjunction of variables and their negations) for the input signals that destroy said bonds, virtually any logic function is realized. Indeed, any logic function can be represented as a complete disjunctive normal form, i.e., as disjunction of conjunctions of variables and their negations. For implementation of the logic function represented in this way, a CLE is used that includes the agents for all "conjunctions", and with the identical blockable labels. Then said CLE implements disjunction of the functions realized by these agents, i.e., if a blockable label is available on at least one of the agents in the CLE, the output action is performed.

**Example 5.** A function, which is a combination of the logic functions YES, NOT and AND (i.e, the conjunction of variables and their negations) for the input signals that mediate the bonds, is realized as follows. The CLE consists, inter alia, of the agent, with which the blockable label binds through the bonds mediated by those input signals, which are comprised in said composite logic function as a value of variable (rather than its negation). Besides, the agent carries a set of the linking receptors 1, which bind with the blocking substance, and for each input signal comprised in said composite logic function with negation, at least one linking receptor 1 exists, the bond of which with the blocking substance is mediated by said input signal and, additionally, for each linking receptor 1 there is an input signal comprised in said composite logic function with negation, which mediates the bond of said linking receptor 1 with the blocking substance.

**Example 6.** Using the CLE shown in Example 6 for implementation of a combination of the logic functions YES, NOT and AND (i.e., the conjunction of variables and their negations) for the input signals that mediate said bonds, virtually any logic function is realized. Indeed, any logic function can be represented as a complete disjunctive normal form, i.e., as disjunction of conjunctions of variables and their negations. For implementation of the logic function represented in this way, a CLE is used that includes the agents for all "conjunctions", and with the identical blockable labels. Then said CLE implements disjunction of the functions realized by these agents, i.e., if a blockable label is available on at least one of the agents in the CLE, the output action is performed. At this, the input signals may be added either before or after mixing of said agents. Besides, these agents may be not mixed with each other, and the output action is considered to be produced if it is produced by at least one said agent after addition of the input signals. The case of the input signals that mediate said bonds is generally more demanding to the order of steps when calculating the value of the logical function than the case of the input signals that destroy said bonds. For example, for realization of the CLE it is preferable to add all the input signals to each agent separately, then add the blockable labels and the blocking substances correspondent to this agent, and then mixing all the agents to produce the final output action. Besides, the addition of the blockable labels and the blocking substances to the agent may be done either before or after addition of the input signals.

The described above examples of CLEs that implement the logical functions are not limited to the cases, in which all the input signals destroy said bonds, or all mediate. These examples are given only for the sake of simplicity and clarity of the description of the invention, it is also possible to implement different logic functions for different combinations of the input signals, some of which destroy, while some of which mediate said bonds; at this, although the implementation of the basic set of functions may differ from the above examples, it remains within the invention.

## Claims

1. A logic element complex that converts input signals into an output action according to a given logic function and comprises at least:
i) an agent (a molecule, a particle, surface of a solid phase) having at least a linking receptor 1 and a linking receptor 2,
ii) a blockable label, participating directly or indirectly in producing of at least one output action and capable of binding directly or indirectly with said linking receptor 2 of said agent so that the binding of said blockable label with said linking receptor 2 of said agent is governed by at least one input signal,
iii) a blocking substance, which is capable of binding directly or indirectly with said linking receptor 1 of said agent depending on at least one of said input signals, and such that upon binding of said blocking substance with said linking receptor 1 of said agent, said blockable label, which under respective input signals is bound with said agent, becomes blocked spatially or spatially-electrostatically, and this blocking causes a change in said output action.

2. A logic element complex according to Claim 1, wherein said output action is produced by said blockable label of said agent depending on direct or indirect interaction of said blockable label with an object, said output action being different from binding of the agents with each other.

3. A logic element complex according to Claim 1, wherein said output action is produced by said blockable label of said agent depending on direct or indirect interaction of said blockable label with either a surface of a solid phase (including a surface of a cell) or an object that produces said output action or changes the output action produced by the blockable label, the object being different from the agent (or its analog).

4. A logic element complex according to Claim 1, wherein at least one of said agent or said blocking substance is a particle selected of magnetic, fluorescent, protein (including cross-linked protein), polymer (polystyrene, dextran, polypeptide, etc.) or crystalline (gold, silver, semiconductor, etc.) nanoparticle/microparticle.

5. A logic element complex according to Claim 1, wherein said blockable label is an enzyme.

6. A logic element complex according to Claim 1, wherein said blocking of said blockable label causes suppression (reduction), at least partial, of said output action.

7. A logic element complex according to Claim 1, wherein at least one of the dependences of binding of the blocking substance or the blockable label with the agent upon the input signal of molecular nature is due to the presence of the "receptor to said input signal - said input signal (or its analog)" bond in a series of bonds between said agent and said blocking substance, or said agent and said blockable label.

8. A logic element complex according to Claim 1, wherein the process of producing of said output action involves specific direct or indirect binding with said blockable label of at least one molecule or particle, which is a label capable of generating the detectible signal, preferably fluorescent, luminescent, enzyme, radioactive, magnetic, or exhibiting surface plasmon resonance properties.

9. A logic element complex according to Claim 8, wherein after binding of said molecule or particle with said blockable label of said agent, a major portion of unbound said molecules or particles is removed (separated), and said output action is produced primarily by said molecules or particles bound with said agent.

10. A logic element complex according to Claim 8, wherein the process of producing of said output action involves specific direct or indirect binding with said blockable label of at least one molecule or particle, immobilized on an auxiliary solid phase (an immunochromatographic test strip, a plastic multi-well plate, etc.), and for this purpose a liquid medium containing said logic element complex is passed along said solid phase or incubated in contact with said solid phase, and said output action is associated with the number of the agents bound with said auxiliary solid phase.

11. A logic element complex according to Claim 1, wherein said output action consists in at least generation of a detectible signal.

12. A logic element complex according to Claim 1, wherein said output action is designated for diagnostics *in vitro* of a disease or condition of a subject.

13. A logic element complex according to Claim 1, wherein said output action is designated for diagnostics *in vivo* of a disease or condition of a subject.

14. A logic element complex according to Claim 1, wherein said output action is designated for therapeutic influence on health or condition of a subject.

15. A logic element complex according to any one of the Claims 1 to 14, which is used as a basis for a pharmaceutical formulation.

16. A logic element complex according to any one of the Claims 12 to 14, wherein at least a part of said input signals is governed by condition of said subject.

17. A logic element complex according to any one of the Claims 12 to 14, wherein the blockable label is a receptor (an antibody to a marker on a cell membrane, a nuclear localization signal peptide, etc.) for targeted delivery of a substance to the area of interest in the organism, a cell, etc.

18. A logic element complex according to any one of the Claims 12 to 14, wherein the output action consists in creation of molecules for improvement of health of the subject or for diagnostics of its (his/her) condition.

19. A logic element complex according to any one of the Claims 12 to 14, which is made intended for administration (intravenously, subcutaneously, etc.) to the subject with the purpose of a disease treatment or diagnostics of condition of the subject.

20. A logic element complex according to any one of the Claims 12 to 14, wherein the output action consists in delivery of a substance to target cells.

21. A logic element complex, which converts input signals into an output action according to a given logic function and comprises at least:
i) an agent (a molecule, a particle, a surface of a solid phase) having at least a linking receptor 1 and a blockable label that participates directly or indirectly in producing at least one output action,
ii) a blocking substance, which is capable of binding directly or indirectly with said linking receptor 1 of said agent depending on at least one of said input signals, and such that upon said binding of said blocking substance with said linking receptor 1 of said agent, said blockable label of said agent becomes blocked spatially or spatially-electrostatically, and this blocking causes a change in said output action,
and said output action is implemented by said blockable label of said agent depending on direct or indirect interaction of said blockable label with an object, said output action being different from binding of the agents with each other.

22. A logic element complex according to Claim 21, wherein said output action is implemented by said blockable label of said agent depending on direct or indirect interaction of said blockable label with either a surface of a solid phase (including a surface of a cell) or an object that produces said output action or changes the output action produced by the blockable label, the object being different from the agent (or its analog).

23. A logic element complex according to Claim 21, wherein at least one of said agent or said blocking substance is a particle selected of magnetic, fluorescent, protein (including cross-linked protein), polymer (polystyrene, dextran, polypeptide, etc.) or crystalline (gold, silver, semiconductor, etc.) nanoparticle/microparticle.

24. A logic element complex according to Claim 21, wherein said blockable label is an enzyme.

25. A logic element complex according to Claim 21, wherein said blocking of said blockable label causes suppression (reduction), at least partial, of said output action.

26. A logic element complex according to Claim 21, wherein at least one of the dependences of binding of the blocking substance or the blockable label with the agent upon the input signal of molecular nature is due to the presence of the "receptor to said input signal - said input signal (or its analog)" bond in a series of bonds between said agent and said blocking substance, or said agent and said blockable label.

27. A logic element complex according to Claim 21, wherein the process of producing of said output action involves a specific direct or indirect binding with said blockable label of at least one molecule or particle, which is a label capable of generating the detectible signal, preferably fluorescent, luminescent, enzyme, radioactive, magnetic, or exhibiting surface plasmon resonance properties.

28. A logic element complex according to Claim 27, wherein after binding of said molecule or particle with said blockable label of said agent, a major portion of unbound said molecules or particles is removed (separated), and said output action is produced primarily by said molecules or particles bound with said agent.

29. A logic element complex according to Claim 28, wherein the process of producing of said output action involves a specific direct or indirect binding with said blockable label of at least one molecule or particle, immobilized on an auxiliary solid phase (an immunochromatographic test strip, a plastic multi-well plate, etc.), and for this purpose a liquid medium containing said logic element complex is passed along said solid phase or incubated in contact with said solid phase, and said output action is associated with the number of the agents bound with said auxiliary solid phase.

30. A logic element complex according to Claim 21, wherein said output action consists in at least generation of a detectible signal.

31. A logic element complex according to Claim 21, wherein said output action is designated for diagnostics *in vitro* of a disease or condition of a subject.

32. A logic element complex according to Claim 21, wherein said output action is designated for diagnostics *in vivo* of a disease or condition of a subject.

33. A logic element complex according to Claim 21, wherein said output action is designated for therapeutic influence on health or condition of a subject.

34. A logic element complex according to any one of the Claims 21 to 33, which is used as a basis for a pharmaceutical formulation.

35. A logic element complex according to any one of the Claims 31 to 33, wherein at least a part of said input signals is governed by a condition of a subject.

36. A logic element complex according to any one of the Claims 31 to 33, wherein the blockable label is a receptor (an antibody to a marker on a cell membrane, a nuclear localization signal peptide, etc.) for targeted delivery of a substance to the area of interest in an organism, a cell, etc.

37. A logic element complex according to any one of the Claims 31 to 33, wherein the output action consists in creation of molecules for enhancement of health of the subject or for diagnostics of its (his/her) condition.

38. A logic element complex according to any one of the Claims 31 to 33, wherein said logic element complex is made intended for administration (intravenously, subcutaneously, etc.) to the subject with a purpose of a disease treatment or diagnostics of condition of the subject.

39. A logic element complex according to any one of the Claims 31 to 33, wherein the output action consists in delivery of a substance to target cells.
